(19) Europäisches Patentamt
European Patent Office
Office européen des brevets

(11) **EP 3 951 035 A1**

(12) **EUROPEAN PATENT APPLICATION**
published in accordance with Art. 153(4) EPC

(43) Date of publication:
**09.02.2022 Bulletin 2022/06**

(21) Application number: **20784324.4**

(22) Date of filing: **30.03.2020**

(51) International Patent Classification (IPC):
*D02G 3/26* (2006.01)     *D04B 1/14* (2006.01)
*A41B 9/06* (2006.01)     *A41D 13/00* (2006.01)
*A41D 13/12* (2006.01)     *A61B 5/0408* (2006.01)
*A61B 5/0478* (2006.01)     *A41D 31/04* (2019.01)

(52) Cooperative Patent Classification (CPC):
**A41B 9/06; A41D 13/00; A41D 13/12; A41D 31/04; A61B 5/25; A61B 5/291; D02G 3/26; D04B 1/14**

(86) International application number:
**PCT/JP2020/014442**

(87) International publication number:
**WO 2020/203945 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2019 JP 2019067964**

(71) Applicants:
• **TOYOBO CO., LTD.**
  **Osaka-shi**
  **Osaka 530-8230 (JP)**
• **TOYOBO STC CO., LTD.**
  **Osaka-shi, Osaka 530-0004 (JP)**

(72) Inventors:
• **SHIMIZU, Yusuke**
  **Otsu-shi, Shiga 520-0292 (JP)**
• **KWON, Euichul**
  **Otsu-shi, Shiga 520-0292 (JP)**
• **MORIMOTO, Shota**
  **Otsu-shi, Shiga 520-0292 (JP)**
• **KOMATSU, Yoko**
  **Otsu-shi, Shiga 520-0292 (JP)**
• **KURODA, Nobuhiro**
  **Osaka-shi, Osaka 530-0004 (JP)**

(74) Representative: **Müller-Boré & Partner**
**Patentanwälte PartG mbB**
**Friedenheimer Brücke 21**
**80639 München (DE)**

(54) **GARMENT**

(57) The purpose of the present invention is to provide a biological information measurement garment having excellent peel strength between a clothing fabric including spun yarns and an electrode. The garment includes a clothing fabric; and an electrode formed on a skin-side surface of the clothing fabric, the clothing fabric including spun yarns present on the skin-side surface on which the electrode is formed, and each of the spun yarns satisfying a following formula (1):

$$F/(0.8673/\sqrt{Ne}) < 4500 \ (1),$$

wherein F represents a number of fluffy fibers (fibers/10 m) having a length of 1 mm or more, per 10 m of the spun yarn, and Ne represents an English yarn count of the spun yarn.

EP 3 951 035 A1

**Description**

TECHNICAL FIELD

The present invention relates to a garment including a clothing fabric and an electrode formed on a skin-side surface of the clothing fabric. In detail, the present invention relates to a garment on which a biological information measuring electrode for detecting biological information of a wearer is formed. Specifically, the present invention relates to a biological information measuring garment having an electrode in direct contact with skin of a wearer, or an electrode to be a detection terminal of a sensor capable of proximally and contactlessly acquiring biological information.

BACKGROUND ART

In recent years, wearable biological information measurement apparatuses (sensing wear) are drawing attention in a health monitoring field, a medical field, an education field, and a rehabilitation field. The wearable biological information measurement apparatuses are apparatuses that include a biological information measurement apparatus provided on, for example, a belt or a strap, and that enables simple measurement of biological information, such as an electrocardiogram, of a person who wears it. As a biological information measurement apparatus, for example, an apparatus, in which a biological information measurement electrode configured to be in contact with skin of a wearer is formed, is known.

In cases of a garment-type wearable biological information measurement apparatus, an electrode is provided on a body clothing fabric formed of, for example, a woven fabric or a knitted fabric, making it possible to simply measure biological information, such as heartbeat variability, in various daily situations of a person who wears the garment and lives daily life.

Various garment-type wearable biological information measurement apparatuses have been known so far, and the inventors of the present invention have proposed, for example in Patent Document 1, a sensing wear that determines a measurement position enabling the most stable measurement of biological information and to which a highly closely contactable flexible electrode is attached.

RELATED ART DOCUMENT

PATENT DOCUMENT

Patent Document 1: JP-A-2017-29692

SUMMARY OF THE INVENTION

PROBLEM TO BE SOLVED BY THE INVENTION

Conventionally, garment-type wearable biological information measurement apparatuses have allowed an electrode thereof to be easily peeled from a clothing fabric when a wearer exercises hard or when the garment is repetitively washed. However, an improvement of peel strength between a clothing fabric including particularly spun yarns and an electrode has not been studied so far. The present invention has been made in view of the circumstances, and an object of the present invention is to provide a biological information measurement garment having excellent peel strength between a clothing fabric including spun yarns and an electrode.

SOLUTIONS TO THE PROBLEM

The garment according to the embodiment of the present invention that has solved the problem has the following configurations.

[1] A garment including:

a clothing fabric; and
an electrode formed on a skin-side surface of the clothing fabric,
the clothing fabric including spun yarns present on the skin-side surface on which the electrode is formed, and
each of the spun yarns satisfying a following formula (1):

$$F/(0.8673/\sqrt{Ne}) < 4500 \ (1),$$

wherein F represents a number of fluffy fibers (fibers/10 m) having a length of 1 mm or more, per 10 m of the spun yarn, and Ne represents an English yarn count of the spun yarn.

[2] The garment according to above [1], having a peel strength between the clothing fabric and the electrode of 7.0 N/cm or more, the peel strength being measured in accordance with JIS L 1086 (2013), 7.10.
[3] The garment according to above [1] or [2], wherein
each of the spun yarns includes 65 mass% or more of cotton.
[4] The garment according to any one of above [1] to [3], including 80 mass% or more of the spun yarns in 100 mass% of the clothing fabric.
[5] The garment according to any one of above [1] to [4], wherein

each of the spun yarns is twisted to have a twist coefficient K of 3.20 or more, the twist coefficient K being obtained by a following formula (2):

$$K = T/\sqrt{Ne} \ (2),$$

wherein K represents the twist coefficient, T represents a number of twists (times/2.54 cm) applied to the spun yarn, and Ne represents the English yarn count of the spun yarn.

[6] The garment according to any one of above [1] to [5], wherein the spun yarns are spun twisted union yarns.
[7] The garment according to any one of above [1] to [6], wherein
the clothing fabric has a basis weight of 150 g/m² or more and 300 g/m² or less.
[8] The garment according to any one of above [1] to [7], wherein
the clothing fabric is a knitted fabric, includes knitted loops present on the skin-side surface thereof, and has, in the knitted loops, a proportion in number of 50% or more of knitted loops formed of the spun yarns.
[9] The garment according to any one of above [1] to [8], wherein
the electrode includes an insulating layer formed on the skin-side surface of the clothing fabric, and a conductive layer formed on the insulating layer.
[10] The garment according to any one of above [1] to [9], being an underwear top.
[11] The garment according to any one of above [1] to [9], being an underwear bottom.
[12] The garment according to any one of above [1] to [9], being a band-shaped fabric.

EFFECTS OF THE INVENTION

According to the present invention, the configurations described above enable provision of biological information measurement garment having excellent peel strength between a clothing fabric including spun yarns and an electrode. Generally, a biological information measurement garment is often produced using, as a base, compression garment including chemical fibers as a main body, due to necessity of bringing an electrode into close contact with skin. However, elderly people or hospitalized patients who are main users in a health monitoring field, a medical field, an education field, and a rehabilitation field are not used to such compression garment and have often complained of discomfort. The garment according to the present invention includes a clothing fabric including soft-texture spun yarns and can therefore reduce the discomfort. In addition, the garment suitably absorbs sweat and the like and therefore enables a wearer to easily avoid discomfort during rehabilitation. Further, the biological information measurement garment including such a soft clothing fabric is skin friendly, leading to reduction of burden on carers.

MODE FOR CARRYING OUT THE INVENTION

A garment according to the present invention includes a clothing fabric and an electrode formed on a skin-side surface of the clothing fabric, the clothing fabric including spun yarns present on the skin-side surface on which the electrode is formed, and each of the spun yarns satisfying a following formula (1).

$$F/(0.8673/\sqrt{Ne}) < 4500 \ (1)$$

(In the formula, F represents a number of fluffy fibers (fibers/10 m) having a length of 1 mm or more, per 10 m of the spun yarn. Ne represents an English yarn count of the spun yarn.)

With the configuration described above, a biological information measurement garment can be provided that has excellent peel strength between a clothing fabric including spun yarns and an electrode. Conventionally, as described in, for example, Patent Document 1, an electrode has been fixed to a clothing fabric by interposing a hot-melt sheet between the clothing fabric and a conductive layer, and heating and melting the sheet and thus making the sheet penetrate into the clothing fabric.

However, according to the study by the inventors of the present invention, it has been found that when spun yarns included in the clothing fabric have a great deal of fluff, sufficient penetration of resin into surfaces of the spun yarns is easily inhibited by the fluff in the fixation of the electrode, not allowing full use of adhesive force of the hot-melt sheet and therefore decreasing the peel strength between the clothing fabric and the electrode. Therefore, the inventors of the present invention have conducted an earnest study and, as a result, found that setting the left-side value in the formula (1) to less than 4500, that is, reducing the number of fluffy fibers of the spun yarns present on the skin-side surface of the clothing fabric to a prescribed amount or less allows the resin to easily penetrate into the clothing fabric and thus enables an improvement of the peel strength between the clothing fabric including spun yarns and the electrode. Such garment having excellent peel strength between the clothing fabric and the electrode can suitably be used as the biological information measurement garment. Hereinafter, each configuration is described in detail.

The garment includes a clothing fabric and an electrode formed on a skin-side surface of the clothing fabric. Direct contact of an electrode surface of the electrode with skin of a wearer enables measurement of electrical signals from a body and thus enables measurement of biological information. As the biological information, information of a body, such as an electrocardiogram, a heart rate, a pulse rate, a breathing rate, blood pressure, body temperature, myopotential, and sweating, can be obtained by an electronic unit calculating and processing the electrical signals acquired through the electrode.

As the electrode, an electrode capable of measuring an electrocardiogram is preferable. The electrocardiogram means information obtained by detecting electrical changes caused by cardiac movement via an electrode on a surface of a living body and recording the changes as a waveform. The electrocardiogram is generally recorded as a waveform formed by plotting the potential difference, with the time put on the horizontal axis and the potential difference on the vertical axis. The waveform that appears every heartbeat on the electrocardiogram is mainly formed of representative five waves, i.e., a P wave, a Q wave, an R wave, an S wave, and a T wave, and there is also a U wave. A part of the start of a Q wave to the end of an S wave is sometimes called a QRS wave. An electrode capable of detecting at least the R wave among these waves is preferable. The R wave represents excitement of both the left and right ventricles and is a wave with the largest potential difference. Providing the electrode capable of detecting the R wave also enables measurement of a heart rate. That is, the time between the top of an R wave and the top of the next R wave is generally called an R-R interval (second), and the heart rate per minute can be calculated on the basis of the following formula. In the present specification, the QRS wave is to be included in the R wave unless otherwise noted. The specific configuration of the electrode is described later in detail.

Heart rate (times/min) = 60/R-R interval

The clothing fabric includes spun yarns present on the skin-side surface on which the electrode is formed, each of the spun yarns satisfying a following formula (1).

$$F/(0.8673/\sqrt{Ne}) < 4500 \ (1)$$

(In the formula, F represents a number of fluffy fibers (fibers/10 m) having a length of 1 mm or more, per 10 m of the spun yarn. Ne represents an English yarn count of the spun yarn.)

Setting the left-side value in the formula (1) to less than 4500, that is, making less fluffy spun yarns present on the skin-side surface of the clothing fabric strengthens the adhesion between the yarns on the surface of the clothing fabric and the electrode and thus enables an easy improvement of the peel strength between the clothing fabric and the electrode. For the improvement, the left-side value in the formula (1) is preferably 4300 or less, further preferably 4200 or less, further more preferably 4000 or less, particularly preferably 3000 or less. On the other hand, setting the left-side value in the formula (1) to 500 or more enables an easy improvement of a heat-retaining property of the clothing fabric. For the improvement, the left-side value in the formula (1) is preferably 500 or more, more preferably 1000 or more, further preferably 2000 or more.

The spun yarn preferably has an English yarn count Ne of 5 or more and 100 or less. The spun yarn having an English yarn count Ne of 5 or more can improve softness of the clothing fabric. For the improvement, the spun yarn has an English yarn count Ne of more preferably 10 or more, further preferably 15 or more. On the other hand, the spun yarn having an English yarn count Ne of 100 or less can easily improve strength of the clothing fabric. For the improvement, the spun yarn has an English yarn count Ne of more preferably 80 or less, further preferably 70 or less. The English yarn count Ne can be measured by the method described later in EXAMPLES.

Each of the spun yarns is preferably twisted to have a twist coefficient K of 3.20 or more, the twist coefficient K being obtained by a following formula (2).

$$K = T/\sqrt{Ne} \ (2)$$

(In the formula, K represents the twist coefficient. T represents a number of twists (times/2.54 cm) applied to the spun yarn. Ne represents an English yarn count of the spun yarn.)

The spun yarn having a twist coefficient K of 3.20 or more easily reduces the fluff and allows the resin to easily go into the clothing fabric when the electrode is formed on the clothing fabric, therefore enabling an easy improvement of the peel strength between the clothing fabric and the electrode. For the improvement, the spun yarn has a twist coefficient K of more preferably 3.40 or more, further preferably 3.60 or more, further more preferably 3.80 or more. On the other hand, the spun yarn having a twist coefficient K of 8.0 or less allows the resin to easily come thereinto when the electrode is formed on the clothing fabric, and therefore enables an easy improvement of the peel strength between the clothing fabric and the electrode. For the improvement, the spun yarn has a twist coefficient K of preferably 8.0 or less, more preferably 7.0 or less, further preferably 6.5 or less.

Each of the spun yarns is preferably at least one selected from the group consisting of, for example, a single yarn obtained by twisting one roving, and a spun twisted union yarn obtained by twisting a plurality of rovings together. Particularly, a spun twisted union yarn allows the resin to easily come thereinto and therefore enables an easy improvement of the peel strength between the clothing fabric and the electrode. Therefore, the spun yarn is more preferably a spun twisted union yarn.

The spun twisted union yarn is a yarn manufactured by drawing (draft) a fleece (roving) in spinning, then intertwining two or more fleeces (rovings) under application of twists, and winding the yarn around a spinning bobbin. In a process of manufacturing the spun twisted union yarn, intertwining fleeces aligned in parallel enables two fiber groups to be twisted together while the parallel degree of the fibers is retained, and thus more easily reduces fluff appearing on a surface of the spun yarn than a single yarn or a two folded yarn. For example, when a single yarn is twisted together alone at a particularly high twist rate, a difference in the twist angle between a center portion and a side peripheral portion of the yarn is easily generated, and in order to relax this distortion, migration of individual fibers in the yarn that is attributed to twisting occurs and thus allows fluff to easily stick out. On the other hand, application of twists that is performed by twisting together two or more fleeces (rovings) with a high parallel degree makes the migration less likely to occur and thus enables the number of fluffy fibers to be easily reduced.

Fibers that form the spun yarn preferably have an effective fiber length of 20 mm or more and 80 mm or less. Such fibers enable easy attainment of both the improvement of the peel strength between the clothing fabric and the electrode and the improvement of the heat-retaining property of the clothing fabric. The fibers have an effective fiber length of more preferably 24 mm or more, further preferably 26 mm or more, and more preferably 60 mm or less, further preferably 40 mm or less. The effective fiber length can be measured by the method described later in EXAMPLES.

When including cotton, each of the spun yarns preferably includes at least extra long staple cotton having an effective fiber length of 38 mm or more. The spun yarn includes more preferably 20 mass% or more of the extra long staple cotton, further preferably 30 mass% or more, further more preferably 40 mass% or more, particularly preferably 60 mass% or more, most preferably 100 mass%. The spun yarn that includes raw cotton having an effective fiber length of 38 mm or more can reduce the fluff on the surface thereof, and has uniform thickness and thus allows an adhesive to uniformly penetrate thereinto, facilitating increase of the peel strength. Examples of the raw cotton having an effective fiber length of 38 mm or more include sea island cotton, Suvin cotton, Giza 45 and 70, Chinese Xinjiang cotton, and Pima cotton.

Examples of the fibers included in the spun yarn include natural fibers, synthetic fibers, and recycled fibers. These types of fibers may be used alone, or two or more types thereof may be used. Among these types of fibers, natural fibers are preferable.

Examples of the natural fibers include cotton, hemp, wool, and silk. Among these types of natural fibers, cotton is preferable. The natural fibers may be used directly or after post processing such as a hydrophilic treatment or a soil-resistance treatment. Examples of the synthetic fibers include polyesters such as acrylic, polyethylene terephthalate, polyethylene naphthalate, and polylactic acid; and polyamides such as nylon 6 and nylon 66. Examples of the recycled fibers include rayon and acetate. These types of fibers may be used alone, or two or more types thereof may be used.

The spun yarn preferably includes 65 mass% or more of the natural fibers. The spun yarn (100 mass%) including 65 mass% or more of the natural fibers enables an easy improvement of the heat-retaining property. For the improvement, the spun yarn includes more preferably 80 mass% or more of the natural fibers, further preferably 90 mass% or more, further more preferably 95 mass% or more, particularly preferably 98 mass% or more, most preferably 100 mass%.

Each of the spun yarns preferably includes 65 mass% or more of cotton. The spun yarn (100 mass%) including 65 mass% or more of cotton enables an easy improvement of a hygroscopic property, a water-absorption property, the heat-retaining property, an aeration property, and the like. In addition, the spun yarn having a high blending proportion of cotton enables a remarkably easy improvement of pilling performance when the formula $F/(0.8673/\sqrt{Ne})$ is set to less than 4500, and such a spun yarn is less likely to generate static electricity and can thus easily reduce noise of biological information signals. Further, such a spun yarn allows cotton to absorb sweat and thus makes the clothing fabric increase force to closely contact with skin, easily improving measurement accuracy during exercise. For the improvement, the spun yarn includes more preferably 80 mass% or more of cotton, further preferably 90 mass% or more, further more preferably 95 mass% or more, particularly preferably 98 mass% or more, most preferably 100 mass%.

The clothing fabric (100 mass%) preferably includes 80 mass% or more of the spun yarns. The clothing fabric (100 mass%) including 80 mass% or more of the spun yarns enables an easy improvement of the heat-retaining property. For the improvement, the clothing fabric includes more preferably 90 mass% or more of the spun yarns, further preferably 95 mass% or more, particularly preferably 98 mass% or more, most preferably 100 mass%.

The clothing fabric may include filament yarns other than the spun yarns. Examples of the filament yarns include monofilament yarns and multifilament yarns. Specific examples of the filament yarns include polyester-based multifilament yarns and polyurethane elastic yarns.

The clothing fabric preferably has a basis weight of 150 g/m$^2$ or more and 300 g/m$^2$ or less. The clothing fabric having a basis weight of 150 g/m$^2$ or more easily improves the strength. For the improvement, the clothing fabric has a basis weight of more preferably 160 g/m$^2$ or more, further preferably 170 g/m$^2$ or more. On the other hand, the clothing fabric having a basis weight of 300 g/m$^2$ or less can easily reduce the weight. For the reduction, the clothing fabric has a basis weight of more preferably 280 g/m$^2$ or less, further preferably 270 g/m$^2$ or less. The basis weight of the clothing fabric can be measured by the method described later in EXAMPLES.

The clothing fabric is preferably a knitted fabric or a woven fabric. The knitted fabric is preferably a weft knitted fabric or a warp knitted fabric, and a weft knitted fabric is preferable. The weft knitted fabric includes a circular knitted fabric.

Examples of the weft knitted fabric (circular knitted fabric) include fabrics having stitch structures such as jersey stitch (plain stitch), bare jersey stitch, welt jersey stitch, fraise stitch (rib stitch), pearl stitch, half tubular stitch, interlock stitch, tuck stitch, float stich, half cardigan stitch, lace stitch, and plating stitch. Among these types of stich, jersey stitch, fraise stich, or interlock stich is preferable, and jersey stich or interlock stich is more preferable. These stitch structures give a flat structure at least on one surface of the fabric and therefore enable an easy improvement of the peel strength between the clothing fabric and the electrode.

Examples of the warp knitted fabric include fabrics having stitch structures such as single denbigh stitch, open-loop denbigh stitch, single atlas stitch, double cord stitch, half stitch, half base stitch, satin stitch, tricot stitch, half tricot stitch, raschel stitch, and jacquard stitch.

The clothing fabric is preferably a knitted fabric that includes knitted loops present on the skin-side surface thereof and that has, in the knitted loops, a proportion in number of 50% or more of knitted loops formed of the spun yarns. The clothing fabric having this structure can easily improve the heat-retaining property on the skin-side surface thereof. For the improvement, the clothing fabric has, in the knitted loops, a proportion in number of more preferably 70% or more of knitted loops formed of the spun yarns, further preferably 80% or more, further more preferably 90% or more, particularly preferably 95% or more.

The number of knitted loops may be counted by raveling a complete structure of the knitted fabric or by observing a surface of the complete structure with a microscope. The structure of the knitted fabric is formed by repetition of one sectional unit structure, and the complete structure refers to the one sectional unit structure.

The woven fabric is not particularly limited, and examples thereof include plain weave, twill weave, sateen weave, multiple weave, dobby weave, and jacquard weave. The fabric may be formed as a fabric with a pattern such as a stripe or a check, using a plurality of types of differently dyed yarns, or may be a patterned woven fabric using a jacquard weaving machine. As a woven fabric particularly used for application of clothing in direct contact with skin, such as a shirt clothing fabric and a blouse clothing fabric, plain weave and twill weave are preferable. In order to improve the peel strength between the clothing fabric and the electrode, a structure having less projections and recesses and less yarn floats on the skin-side surface is preferred, and therefore plain weave is more preferable.

The clothing fabric including the spun yarns (hereinafter, sometimes called a spun yarn-containing clothing fabric) has heretofore been described. The spun yarn-containing clothing fabric can be preferably used not only for an electrode-formed part of the garment, but also for a region other than the electrode-formed part. That is, the garment preferably has, in the skin-side surface (100 area%), an area proportion of the spun yarn-containing clothing fabric on the skin-side surface of preferably 20 area% or more, more preferably 50 area% or more, further preferably 90 area% or more, further more preferably 95 area% or more. The garment can include a different known clothing fabric in the region other than the spun yarn-containing clothing fabric.

The form of the garment is not particularly limited as long as it allows formation of the electrode, and is preferably underwear, a band-shaped fabric, or the like. In addition, the garment may be a fabric that covers at least any one of a breast, a hand, a leg, a foot, a neck, and a face.

As the underwear, an underwear top or an underwear bottom is preferable. Examples of the underwear top include a T-shirt, a polo shirt, a camisole, a brassiere, sports innerwear, a hospital gown, and pajamas. Examples of the underwear bottom include pants, sports innerwear, a hospital gown, and pajamas.

Examples of the band-shaped fabric include a belt, and specific examples thereof include a breast belt and an abdominal belt.

The garment preferably has a peel strength between the clothing fabric and the electrode of 7.0 N/cm or more, the peel strength being measured in accordance with JIS L 1086 (2013), 7.10. The garment that includes the electrode firmly fixed to the clothing fabric as described above enable accurate and easy measurement of biological information and can therefore be suitably used as the biological information measurement garment. The garment has a peel strength of preferably 7.5 N/cm or more, more preferably 8.0 N/cm or more, further preferably 8.5 N/cm or more, further more preferably 10 N/cm or more. The upper limit of the peel strength is not particularly limited, and may be, for example, 20 N/cm or 15 N/cm.

Next, the electrode provided on the skin-side surface of the clothing fabric is described. The electrode is used for detecting a biological potential mainly through contact with skin, but may be used as an electric contact of a connector or the like or may be used as a detection terminal of another proximate and contactless sensor. The electrode is preferably stretchable so as to be capable of following exercise motion of a subject. Examples of the stretchable electrode include an electrode formed of a conductive structure, and a sheet-shaped electrode formed from a conductive composition containing a conductive filler and stretchable resin. As the electrode formed of a conductive structure, it is possible to use, for example, a conductive fiber or a conductive yarn obtained by covering a base fiber with a conductive polymer, a fiber having a surface thereof covered with a conductive metal such as silver, gold, copper, or nickel, a conductive yarn made of a conductive fine metal wire, conductive yarns obtained by mixed spinning of a conductive fine metal wire and a non-conductive fiber, a woven fabric, a knitted fabric, or a nonwoven fabric made of the conductive fiber or the conductive yarn, or a non-conductive fabric embroidered using these conductive yarns.

Using, as a material for the sheet-shaped electrode, for example, a conductive filler having high conductivity enables lowering the electric resistance value compared to the use of a fibrous electrode and thus enables detection of faint electrical signals.

The electrode preferably includes an insulating layer formed on the skin-side surface of the clothing fabric, and a conductive layer formed on the insulating layer. As the insulating layer, a first insulating layer described later is preferable. The insulating layer may be formed directly on the skin-side surface of the clothing fabric, but is preferably bonded and fixed to the skin-side surface of the clothing fabric, with an adhesive layer (described later) interposed between the insulating layer and the skin-side surface. The electrode may consist of the conductive layer without the insulating layer. In this case, the conductive layer may be formed directly on the skin-side surface of the clothing fabric, but is preferably bonded and fixed to the skin-side surface of the clothing fabric, with an adhesive layer (described later) interposed between the conductive layer and the skin-side surface. Further, the garment preferably includes, in addition to the electrode, wiring connecting the electrode to an electronic unit or the like having a function of calculating electrical signals acquired through the electrode. The wiring preferably includes the first insulating layer formed on the skin-side surface of the clothing fabric, the conductive layer formed on a skin-side surface of the first insulating layer, and a second insulating layer formed on a skin-side surface of the conductive layer.

Hereinafter, the conductive layer, the first insulating layer, and the second insulating layer are specifically described.

(Conductive layer)

The conductive layer is something capable of detecting electrical information of a living body and is necessary to secure the conduction. The conductive layer preferably includes a conductive filler and stretchable resin, more preferably includes a conductive filler and elastomer. The conductive layer can be formed using a composition (hereinafter, sometimes referred to as a conductive paste) obtained by dissolving or dispersing components in an organic solvent.

As the conductive filler, it is possible to use, for example, a metal powder, metal nanoparticles, and a conductive material other than the metal powder. A single type of material, or two or more types of materials may be used as the conductive filler. Examples of the metal powder include noble metal powders such as a silver powder, a gold powder, a platinum powder, and a palladium powder; base metal powders such as a copper powder, a nickel powder, an aluminum powder, and a brass powder; a plated powder obtained by plating different types of particles made of inorganic substances such as a base metal and silica with a noble metal such as silver; and an alloyed base metal powder obtained by alloying a base metal and a noble metal such as silver. Among these materials, a silver powder and/or a copper is preferable and enables the conductive layer to exhibit high conductivity at low costs. The silver powder and/or the copper powder is preferably a main component of the metal powder used as the conductive filler, and the main component means a total of 50 mass% or more. Examples of the metal nanoparticles include particles having a particle size of several to several tens of nanometers among the metal powders described above.

The conductive filler has a proportion of the metal powder of preferably 20 vol% or less, more preferably 15 vol% or less, further preferably 10 vol% or less. The conductive filler having an excessively large content proportion of the metal powder sometimes has difficulty being uniformly dispersed in resin, and the metal powders described above are generally expensive. Therefore, it is preferable to suppress the use amount of the metal powder to the above range. The conductive filler has a proportion of the metal nanoparticles of preferably 20 vol% or less, more preferably 15 vol% or less, further preferably 10 vol% or less. The conductive filler having an excessively large content proportion of the metal nanoparticles sometimes has difficulty being uniformly dispersed in resin, and the metal nanoparticles described above are generally expensive. Therefore, it is preferable to suppress the use amount of the metal nanoparticles to the above range.

Examples of the conductive material other than the metal powder include carbon-based materials such as graphite, carbon black, and carbon nanotube. The conductive material other than the metal power preferably has a mercapto group, an amino group, or a nitrile group on a surface thereof, or preferably has a surface thereof surface-treated with rubber containing a sulfide bond and/or a nitrile group. Generally, the conductive material other than the metal powder has, by itself, strong agglomeration force, and has bad dispersibility in resin when the conductive material other than the metal powder has a high aspect ratio. However, by having a mercapto group, an amino group, or a nitrile group on the surface, or by being surface-treated with rubber containing a sulfide bond and/or a nitrile group, the conductive material other than the metal powder increases the affinity for resin and is dispersed, enabling formation of an effective conductive network and thus realization of high conductivity. The conductive filler has a proportion of the conductive material other than the metal powder of preferably 20 vol% or less, more preferably 15 vol% or less, further preferably 10 vol% or less. The conductive filler having an excessively large content proportion of the conductive material other than the metal powder sometimes has difficulty being uniformly dispersed in resin, and the conductive materials other than the metal powder that are described above are generally expensive. Therefore, it is preferable to suppress the use amount of the conductive material other than the metal powder to the above range.

As the conductive layer, it is acceptable to use a plurality of conductive layers that are integrated by stacking or aligning two or more types of conductive layers, the two or more types of conductive layers being obtained by changing the type of the conductive filler, the addition amount of the conductive filler, and the like. The conductive layer has a proportion of the conductive filler (in other words, the conductive paste (total solid content) for forming the conductive layer has a proportion of the conductive filler) of preferably 25 mass% or more and 98 mass% or less, more preferably 27 mass% or more and 95 mass% or less, further preferably 30 mass% or more and 90 mass% or less. The conductive layer having an excessively small content of the conductive filler may possibly have insufficient conductivity. On the other hand, the conductive layer having an excessively large content of the conductive filler is likely to decrease the stretchability, and may therefore possibly generate a crack or the like and cannot retain good conductivity when the electrode and the wiring are extended.

The stretchable resin preferably includes at least, for example, rubber containing a sulfur atom and/or rubber containing a nitrile group. The sulfur atom and the nitrile group have high affinity for the conductive filler (particularly, the metal powder), and the rubber is highly stretchable. Therefore, the load per unit width in 10% extension of the electrode and the wiring can be reduced and the generation of a crack or the like in extension can be avoided. In addition, such resin can retain uniform dispersion of the conductive filler even when the electrode and the wiring are extended, and can therefore reduce the variation of the electric resistance in 20% extension and enable the electrode and the wiring to exhibit excellent conductivity. Further, such resin enables the electrode and the wiring to exhibit excellent conductivity even when the thickness of the electrode and the wiring is reduced. Between two types of rubber, rubber containing a nitrile group is more preferable and can furthermore reduce the variation of the electric resistance in 20% extension.

As the rubber containing a sulfur atom, elastomer may be used besides the rubber containing a sulfur atom. The sulfur atom is contained in the form of a sulfide bond or a disulfide bond in the main chain of a polymer, a mercapto group in a side chain or at a terminal, or the like. Examples of the rubber containing a sulfur atom include polysulfide rubber, polyether rubber, polyacrylate rubber, and silicone rubber that contain a mercapto group, a sulfide bond, or a disulfide bond. Particularly, polysulfide rubber, polyether rubber, polyacrylate rubber, and silicone rubber that contain a mercapto group are preferable. As a commercially available product that can be used as the rubber containing a sulfur atom, a preferable example is liquid polysulfide rubber "THIOKOL (registered trademark) LP" manufactured by Toray Fine Chemicals Co., Ltd. The rubber containing a sulfur atom preferably has a content of the sulfur atom of 10 to 30 mass%. As the rubber containing no sulfur atom, it is also possible to use, for example, resin in which a sulfur-containing compound such as pentaerythritol tetrakis(S-mercaptobutyrate), trimethylolpropane tris(S-mercaptobutyrate), or mercapto group-containing silicone oil is blended.

As the rubber containing a nitrile group, elastomer may be used besides the rubber containing a nitrile group. Particularly, an acrylonitrile-butadiene copolymer rubber that is a copolymer of butadiene and acrylonitrile is a preferable example. As a commercially available product that can be used as the rubber containing a nitrile group, preferable examples are Nipol (registered trademark) 1042 and Nipol (registered trademark) DN003 that are manufactured by Zeon Corporation. The rubber containing a nitrile group has an amount of the nitrile group (particularly, the acrylonitrile-butadiene copolymer rubber has an amount of acrylonitrile) of preferably 18 to 50 mass%, more preferably 20 to 45 mass%, further preferably 28 to 41 mass%. Particularly, the acrylonitrile-butadiene copolymer rubber having an excessively large amount of bonded acrylonitrile increases the affinity for the conductive filler, particularly the metal powder, but inversely reduces the rubber elasticity contributing to the stretchability.

A single type of resin, or two or more types of resin may be used as the stretchable resin that forms the conductive layer. That is, the stretchable resin that forms the conductive layer is preferably formed of only the rubber containing a sulfur atom and the rubber containing a nitrile group, but may contain stretchable resin other than the rubber containing a sulfur atom and the rubber containing a nitrile group in a range not to impair, for example, conductivity, stretchability, coatability during forming the conductive layer. When including another type of stretchable resin, the entire resin has a total amount of the rubber containing a sulfur atom and the rubber containing a nitrile group of preferably 95 mass% or more, more preferably 98 mass% or more, further preferably 99 mass% or more. The conductive layer has a proportion of the stretchable resin (in other words, the conductive paste (total solid content) for forming the conductive layer has a proportion of the stretchable resin (solid content)) of preferably 2 mass% or more and 75 mass% or less, more preferably 5 mass% or more and 73 mass% or less, further preferably 10 mass% or more and 70 mass% or less. The conductive layer having an excessively small content of the stretchable resin has high conductivity but is likely to deteriorate the stretchability. On the other hand, the conductive layer having an excessively large content of the stretchable resin has good stretchability but is likely to decrease the conductivity.

The conductive layer can be formed directly on the first insulating layer described later, using a composition (conductive paste) obtained by dissolving or dispersing the components described above in an organic solvent, or can be formed by forming a coating film in a desired pattern through coating or printing, and volatilizing the organic solvent included in the coating film and thus drying the coating film. The conductive layer may also be formed by applying the conductive paste to or performing printing with the conductive paste on a release sheet or the like to form a coating film, volatilizing the organic solvent included in the coating film and thus drying the coating film to form a sheet-shaped conductive layer in advance, and stacking the conductive layer in a desired pattern on the first insulating layer described later. The conductive paste may be prepared employing a conventionally known method for dispersing a powder substance in a liquid, and can be prepared by uniformly dispersing the conductive filler in the stretchable resin. The conductive paste may be prepared by mixing, for example, the metal powder, the metal nanoparticles, or the conductive material other than the metal powder with a resin solution, and then uniformly dispersing the contents by an ultrasonic method or with a mixer, a triple ball mill, or a ball mill. A plurality of these types of means can be used in combination. The method for applying the conductive paste or performing printing with the conductive paste is not particularly limited, and a printing method can be employed, such as coating, screen printing, litho offset printing, ink-jet printing, flexo printing, gravure printing, gravure offset printing, stamping, dispensing, or squeegee printing.

The conductive layer has a dried film thickness of preferably 10 to 150 $\mu$m, more preferably 20 to 130 $\mu$m, further preferably 30 to 100 $\mu$m. The conductive layer having an excessively small dried thickness leads to easy deteriorate of the electrode and the wiring due to repetitive stretch and contraction and may thus possibly inhibit or block the conduction. On the other hand, the conductive layer having an excessively large dried film thickness impairs the stretchability, and makes the electrode and the wiring excessively thick and may thus possibly deteriorate wear comfort.

(First insulating layer)

The first insulating layer acts as an insulating layer, and also acts as a waterproof layer that prevents water from reaching the conductive layer, the water coming from the side opposite from the side, on which the first insulating layer is stacked, of the clothing fabric of the garment worn (that is, the outer side of the garment). The first insulating layer may be adhesive. In addition, the first insulating layer provided on the garment side of the conductive layer can suppress stretch of the clothing fabric and thus prevent the conductive layer from being excessively extended. As a result, the first insulating layer can be prevented from generating a crack. Contrarily, although the conductive layer has good extensibility as described above, the conductive layer which is directly formed on the surface of the clothing fabric is excessively stretched following the stretch of the clothing fabric when the clothing fabric is a material having a good stretch property exceeding the extensibility of the conductive layer. The conductive layer is considered to generate a crack as a result of this phenomenon.

The first insulating layer may be formed of insulating resin, and the type of resin is not particularly limited. As the resin, it is possible to preferably use, for example, polyurethane-based resin, silicone-based resin, vinyl chloride-based resin, epoxy-based resin, and polyester elastomer. Among these types of resin, polyurethane-based resin is more preferable, and has further good adhesiveness between the conductive layer and the clothing fabric. A single type of resin, or two or more types of resin may be used as the resin forming the first insulating layer. In addition, the first insulating layer is not limited to one layer but may be two layers. The method for forming the first insulating layer is not particularly limited, and can be formed, for example, by dissolving or dispersing the insulating resin in a solvent (preferably water), applying the resulting solution to or performing printing with the resulting solution on release paper or a release film to form a coating film, and volatilizing the solvent included in the coating film and thus drying the coating film. A commercially available resin sheet or resin film can also be used.

The first insulating layer preferably has an average film thickness of 10 to 200 $\mu$m. The first insulating layer that is excessively thin sometimes has an insufficient insulating effect and an insufficient stretch preventing effect. Accordingly, the first insulating layer has an average film thickness of preferably 10 $\mu$m or more, more preferably 30 $\mu$m or more, further preferably 40 $\mu$m or more. However, the first insulating layer that is excessively thick, sometimes impairs the stretchability of the electrode and the wiring. In addition, the first insulating layer that is excessively thick makes the electrode and the wiring excessively thick and may thus possibly deteriorate wear comfort. Accordingly, the first insulating layer has an average film thickness of preferably 200 $\mu$m or less, more preferably 180 $\mu$m or less, further preferably 150 $\mu$m or less.

The first insulating layer preferably has, in 100 area% of a front side surface opposite from the skin-side surface thereof, a proportion of 40 area% or more of a region fixed to the skin-side surface of the spun yarn-containing clothing fabric. The first insulating layer has a proportion of the region of more preferably 60 area% or more, further preferably 80 area% or more, further more preferably 90 area% or more, particularly preferably 95 area% or more, most preferably 100 area%.

(Second insulating layer)

The wiring preferably includes a second insulating layer formed on the conductive layer. The second insulating layer provided can prevent the conductive layer from contacting with, for example, water such as rain, snow, and sweat. Examples of resin forming the second insulating layer include the same types of resin as the above-described resin forming the first insulating layer, and preferably used resin is also the same. A single type of resin, or two or more types of resin may also be used as the resin forming the second insulating layer. The resin forming the second insulating layer may be the same as or different from the resin forming the first insulating layer, but is preferably the same. The use of the same resin can reduce damage on the conductive layer caused by bias in coverage of the conductive layer and in stress during stretch or contraction of the wiring. The second insulating layer can be formed by the same forming method as the first insulating layer. A commercially available resin sheet or resin film can also be used.

The second insulating layer preferably has an average film thickness of 10 to 200 $\mu$m. The second insulating layer that is excessively thin is easily deteriorated when repetitively stretched and contracted, and thus sometimes has an insufficient insulating effect. Accordingly, the second insulating layer has an average film thickness of preferably 10 $\mu$m or more, more preferably 30 $\mu$m or more, further preferably 40 $\mu$m or more. However, the second insulating layer that is excessively thick, impairs the stretchability of the wiring, and makes the wiring excessively thick and may thus possibly deteriorate wear comfort. Accordingly, the second insulating layer has an average film thickness of preferably 200 $\mu$m or less, more preferably 180 $\mu$m or less, further preferably 150 $\mu$m or less.

(Adhesive layer)

As described above, the electrode is preferably bonded and fixed to the clothing fabric, with an adhesive layer interposed between the electrode and the clothing fabric. The adhesive layer can be formed, for example, by an adhesive. As the adhesive, it is possible to use, for example, a urea resin-based adhesive, a melamine resin-based adhesive, a phenolic resin-based adhesive, a solvent-type adhesive, a water-based adhesive, a reaction-type adhesive, and a hot-melt adhesive. These types of adhesives may be used alone, or two or more types thereof may be used. Among these adhesives, a hot-melt adhesive is preferable.

As the method for applying the adhesive, for example, powder coating, spray coating, coating, printing, or a heat treatment, pressure bonding, or the like after attachment of an adhesive sheet can be performed to bond the clothing fabric with the electrode, the clothing fabric with the insulating layer, or the like.

As the hot-melt adhesive, it is possible to use a polyethylene-based adhesive, a polyamide-based adhesive, a soft polyvinyl chloride-based adhesive, a polyvinyl acetate-based adhesive, a polyester-based adhesive, a thermoplastic polyurethane-based resin, and the like. Among these hot-melt adhesives, a thermoplastic polyurethane-based resin is preferable, because it is highly soft and can maintain high softness around the electrode after bonding. As to the form of the hot-melt adhesive, the hot-melt adhesive in various forms, such as a sheet shape, a powder, and a liquid, can be used. Among these forms, a sheet-shaped hot-melt adhesive is preferable, because it facilitates improvement of the peel strength between the electrode and the clothing fabric. The same material may be used for the insulating layer and the adhesive layer.

As the solvent-type adhesive, it is possible to use a vinyl acetate resin-based solvent-type adhesive, a rubber-based solvent-type adhesive, and other resin-based adhesives. As the water-based adhesive, it is possible to use an EVA resin-based emulsion-type adhesive, an acrylic resin-based emulsion-type adhesive, a vinyl acetate resin-based emulsion-type adhesive, a vinyl acetate copolymerized resin-based and-type adhesive, and the like. As the reaction-type adhesive, it is possible to use, an epoxy resin-based adhesive, a cyanoacrylate-based adhesive, a polyurethane-based adhesive, an acrylic resin-based adhesive, and the like.

The electrode and the wiring preferably have a load per unit width in 10% extension of 100 N/cm or less. When having a load per unit width in 10% extension of more than 100 N/cm, the electrode and the wiring have difficulty making the extension thereof follow the extension of the clothing fabric and thus sometimes impair wear comfort of the garment worn. Accordingly, the electrode and the wiring have a load per unit width in 10% extension of preferably 100 N/cm or less, more preferably 80 N/cm or less, further preferably 50 N/cm or less.

The electrode and the wiring preferably have a variation of electric resistance in 20% extension of 5 times or less. The electrode and the wiring that have a variation of electric resistance in 20% extension of more than 5 times remarkably decrease the conductivity. Accordingly, the electrode and the wiring have a variation of electric resistance in 20% extension of preferably 5 times or less, more preferably 4 times or less, further preferably 3 times or less.

The electrode and the wiring may be formed of different materials, but are preferably formed of the same material. When the electrode and the wiring are formed of the same material, the wiring is set to have a width of preferably 1 mm or more, more preferably 3 mm or more, further preferably 5 mm or more. The upper limit of the wiring width is not particularly limited, but is preferably set to, for example, 10 mm or less, more preferably 9 mm or less, further preferably 8 mm or less.

The electrode and the wiring are preferably formed directly on the clothing fabric forming the garment. The method for forming the electrode and the wiring on the clothing fabric is not particularly limited as long as it does not prevent establishment of the stretchability of the electrode and the wiring. From the viewpoints of a good fit of the garment worn and the following property of the garment in exercise and motion, for example, a known method can be employed, such as stacking with an adhesive or stacking by heat pressing.

The electrode is preferably provided in a chest part or an abdominal part under the chest of the garment. The electrode provided in the chest part or the abdominal part under the chest of the garment enables accurate measurement of biological information. The electrode is more preferably provided, on the garment, in a region that contacts with skin between the upper end of the seventh rib and the lower end of the ninth rib of a wearer. The electrode is preferably provided, on the garment, in a ventral region of a wearer between lines that are parallel with left and right posterior axillary lines of the wearer and are drawn at positions 10 cm backward away from the posterior axillary lines of the wearer. The electrode is preferably provided circularly along the waistline of the wearer. The garment is provided with at least two electrodes, and the two electrodes are preferably provided in a chest part or an abdominal part under the chest of the garment, and the two electrodes are preferably provided in a ventral region of a wearer between lines that are parallel with left and right posterior axillary lines of the wearer and are drawn at positions 10 cm backward away from the posterior axillary lines of the wearer. When three or more electrodes are provided, the positions at which the third and following electrodes are provided are not particularly limited, and may be provided, for example, on the clothing fabric of a back body.

The electrode has an electric resistance value on an electrode surface of preferably 1000 $\Omega$/cm or less, more preferably 300 $\Omega$/cm or less, further preferably 200 $\Omega$/cm or less, particularly preferably 100 $\Omega$/cm or less. Particularly, the electrode having a sheet shaped-form can usually suppress the electric resistance on the electrode surface to 300 $\Omega$/cm or less.

The form of the electrode is preferably a sheet shape. The electrode formed in a sheet shape can have a wide electrode surface and can therefore secure the contact area with skin of a wearer. The sheet-shaped electrode preferably has good flexibility. The sheet-shaped electrode is also preferably stretchable. The size of the sheet-shaped electrode is not particularly limited as long as it can measure electrical signals from a body. The electrode preferably has an area of the electrode surface of 5 to 100 cm$^2$ and preferably has an average thickness of 10 to 500 $\mu$m. The electrode has an area of the electrode surface of more preferably 10 cm$^2$ or more, further preferably 15 cm$^2$ or more. The electrode has an area of the electrode surface of more preferably 90 cm$^2$ or less, further preferably 80 cm$^2$ or less. The electrode that is excessively thin sometimes has insufficient conductivity. Accordingly, the electrode has an average thickness of preferably 10 $\mu$m or more, more preferably 30 $\mu$m or more, further preferably 50 $\mu$m or more. However, the electrode that is excessively thick sometimes gives a wearer a feeling of a foreign body and thus discomfort. Accordingly, the electrode has an average thickness of preferably 500 $\mu$m or less, more preferably 450 $\mu$m or less, further preferably 400 $\mu$m or less. The shape of the electrode is not particularly limited as long as it goes along the curve of a body corresponding to the position at which the electrode is disposed and easily allows the electrode to follow the movement of the body and closely contact with the body. Examples of the shape include a tetragon, a triangle, a five or more sided polygon, a circle, and an ellipse. When the electrode has a polygonal shape, the vertices of the polygon may be rounded not to allow a scratch on skin.

The wiring may be formed using a conductive fiber or a conductive yarn. As the conductive fiber or the conductive yarn, it is possible to use, for example, one obtained by plating a surface of an insulating fiber with a metal, one obtained by twisting a fine metal wire in a yarn, one obtained by impregnating a conductive polymer into between fibers such as microfibers, and a fine metal wire. The wiring preferably has an average thickness of 10 to 500 $\mu$m. The wiring having an excessively small thickness sometimes has insufficient conductivity. Accordingly, the wiring has an average thickness of preferably 10 $\mu$m or more, more preferably 30 $\mu$m or more, further preferably 50 $\mu$m or more. However, the wiring having an excessively large thickness sometimes gives a wearer a feeling of a foreign body and thus discomfort. Accordingly, the wiring has an average thickness of preferably 500 $\mu$m or less, more preferably 300 $\mu$m or less, further preferably 200 $\mu$m or less. The shape of the wiring is not particularly limited and may be a redundant geometrical pattern as well as a straight line and a curve. Examples of the redundant geometrical pattern include a zigzag shape, a continuous horseshoe shape, and a wave shape. The electrode in the redundant geometrical pattern can be formed using, for example, a metal foil.

The garment preferably includes an electronic unit or the like having a function of calculating electrical signals acquired through the electrode. The electronic unit or the like calculates and processes electrical signals acquired through the electrode and thus gives, for example, biological information such as an electrocardiogram, a heart rate, a pulse rate, a breathing rate, blood pressure, body temperature, myopotential, and sweating.

The garment preferably includes, on a front side surface opposite from the skin-side surface of the clothing fabric, a fastener used for connection to the electronic unit. The fastener is a so-called hook, and examples thereof include a stainless-steel hook. The conductive layer and the electronic unit can be electrically connected to each other via the fastener.

The electronic unit or the like is preferably detachable from the garment. Further, the electronic unit or the like preferably includes display means, storage means, communication means, a USB connector, and the like. The electronic unit or the like may also include, for example, a sensor capable of measuring environmental information such as atmospheric temperature, humidity, or atmospheric pressure, or a sensor capable of measuring positional information using a GPS.

Using the garment also enables application to a technique of grasping a human psychological or physiological state. For example, the degree of relaxation can be detected for mental training, sleepiness can be detected for preventing drowsy driving, or an electrocardiogram can be measured for diagnosis of depression, stress, or the like.

The present application claims priority based on Japanese Patent Application No. 2019-067964 filed on March 29, 2019. All the contents described in Japanese Patent Application No. 2019-067964 filed on March 29, 2019 are incorporated herein by reference.

EXAMPLES

Hereinafter, the present invention is more specifically described by way of examples. The present invention is not limited by the following examples, and can be modified and implemented within the scope of complying with the gist of the descriptions above and below. Those all fall within the technical scope of the present invention.

Effective fiber length: In accordance with 7.2.1 Fiber length, Method A (double sorter method) of JIS L 1019: 2006, the effective fiber length of the fibers forming the spun yarns was measured.

Number of fluffy fibers (fibers/10 m): In accordance with 9.22 Method 2B of JIS L 1095, a one-way parallel beam was perpendicularly applied to the spun yarn to project a shadow image of fluff on a shielding plate placed on a side opposite from a light source across the spun yarn, and the number of projected fluffy fibers having a length of 1 mm or more was measured. Specifically, the number of fluffy fibers having a length of 1 mm or more was measured 25 times using F-INDEX TESTER manufactured by Shikishima Spinning Co., Ltd., under the conditions of the length of the spun yarn: 10 m and the yarn speed: 30 m/min, and the average value of the measurement was calculated.

Number of twists and twist coefficient: In accordance with 9.15 Method A of JIS L 1095:2010, the number of twists of the spun yarn was measured, and substituted in the formula (2) to obtain the twist coefficient (K).

English yarn count: In accordance with 9.4.2 of JIS L 1095 (2010), the apparent cotton yarn count of the spun yarn was measured and defined as the English yarn count.

Basis weight: In accordance with "Mass per unit area in standard state" specified in 8.3.2 of JIS L 1096 (2010), the basis weight of the clothing fabric was measured.

Peel strength: On the basis of 7.10 Peel strength of JIS L 1086 (2013) Testing methods for fusible interlining fabrics and laminated fabrics, the peel strength of the following stretchable electrode parts and the clothing fabrics was measured.

Moisture absorptivity: In accordance with 8.10 Moisture percentage and water content ratio of JIS L 1096 (2010), the moisture percentage of the clothing fabric was measured and defined as moisture absorptivity.

Frictional electrostatic voltage: In accordance with 7.2 Method B of JIS L 1094 (2014), the frictional electrostatic voltage of the clothing fabric was measured.

Pilling: In accordance with 7.1 Method A (method using ICI tester) of JIS L 1076 (2012), the pilling of the clothing fabric was measured. The knitted fabric was subjected to operation for 5 hours.

Example 1

Rovings having a mass of 160 grains/15 yd were produced using a total 100 mass% of cotton (effective fiber length: 32.5 mm) obtained by blending Giza 88 cotton (effective fiber length: 36 mm) and Australian cotton (average fiber length: 29 mm) at a mass ratio of 50 : 50. The obtained rovings were drafted at 28 times and real-twisted under the condition of the number of twists T: 17.5 times/2.54 cm (twist coefficient K: 3.91) using a ring spinning frame, and thus spun into spun yarns with a yarn count of 20. The number of fluffy fibers having a length of 1 mm or more, per 10 m of the obtained spun yarns, was 792.6 (fibers/10 m). Next, the spun yarns were knitted in jersey stich using 3FA single knitting machine 34"-22G manufactured by Precision Fukuhara Works, Ltd., under the condition of 350 mm/100 W. The obtained knitted gray fabric was subjected to common scouring and bleaching and dyed by a reactive dye, and then finished by a hydrophilic treatment into a clothing fabric. The obtained clothing fabric had a total width of 157 cm and a basis weight of 264 g/m$^2$.

Next, electrodes and wiring were formed on the skin-side surface of the obtained clothing fabric under the following conditions.

(Conductive paste)

Twenty parts by mass of nitrile rubber (Nipol (registered trademark) DN003 manufactured by Zeon Corporation) was dissolved in 80 parts by mass of isophorone to prepare an NBR solution. In 100 parts by mass of the obtained NBR solution were blended 110 parts by mass of silver particles ("agglomerate silver powder G35" manufactured by DOWA Electronics Materials Co., Ltd., average particle size: 5.9 $\mu$m), and the mixture was kneaded by a triple roll mill to give a conductive paste.

(Electrodes and wiring)

The obtained conductive paste was applied onto a release sheet and dried by a hot air dry oven set to 120°C for 30 minutes or more, to produce a release sheet-attached sheet-shaped conductive layer. Next, a polyurethane hot-melt sheet was attached to the conductive layer-side surface of the release sheet-attached sheet-shaped conductive layer and stacked using a hot pressing machine under the conditions of a pressure of 0.5 kgf/cm$^2$, a temperature of 130°C, and a pressing time of 20 seconds, and the release sheet was peeled to give a polyurethane hot-melt sheet-attached sheet-shaped conductive layer (length: 12 cm and width: 2 cm).

Separately, a polyurethane hot-melt sheet having a length of 13 cm and a width of 2.4 cm was prepared, on which the polyurethane hot-melt sheet-attached sheet-shaped conductive layer (length: 12 cm and width: 2 cm) was stacked, with the polyurethane hot-melt sheet side of the conductive layer directed to the polyurethane hot-melt sheet and one ends in the length direction of the sheet and the conductive layer aligned. These polyurethane hot-melt sheets correspond to the first insulating layer.

Next, a second insulating layer was formed on the conductive layer by stacking the same polyurethane hot-melt sheet as the first insulating layer, so as to cover a part of the first insulating layer and the conductive layer, in a region with a length of 5 cm and a width of 2.4 cm, and from a part 2 cm away from the end. That is, a stretchable electrode part was produced in which an electrode (device connection portion), a wiring portion, and an electrode (detection portion) were longitudinally disposed in this order, the electrode (device connection portion) exposing the conductive layer on the one-end side of the stretchable electrode part and having a size of length 2 cm $\times$ width 2 cm, the wiring portion having a stack structure of the first insulating layer/the conductive layer/the second insulating layer, and the electrode (detection portion) exposing the conductive layer on the other-end side and having a size of length 5 cm $\times$ width 2 cm. The obtained stretchable electrode part was attached to the clothing fabric by thermal compression bonding, with a hot-melt sheet (MOB100S manufactured by Nisshinbo Clothing fabric Inc.) as an adhesive layer interposed between the stretchable electrode part and the clothing fabric, and with the first insulating layer side directed to the skin-side surface of the clothing fabric.

Example 2

Rovings having a mass of 60 grains/15 yd were produced using a total 100 mass% of cotton (effective fiber length: 37.5 mm) obtained by blending Giza 45 cotton (effective fiber length: 39 mm) and Giza 88 cotton (effective fiber length: 36 mm) at a mass ratio of 50 : 50. Two of the obtained rovings were drafted at 42 times and real-twisted under the condition of the number of twists T: 23.3 times/2.54 cm (twist coefficient K: 3.68) using a ring spinning frame and thus spun by spinning and intertwining into spun yarns with a yarn count of 40. The number of fluffy fibers having a length of 1 mm or more, per 10 m of the obtained spun yarns, was 361.7 (fibers/10 m). Next, the spun yarns were knitted in jersey stich using 3FA single knitting machine 34"-28G manufactured by Precision Fukuhara Works, Ltd., under the condition of 250 mm/100 W. The obtained knitted gray fabric was subjected to common scouring and bleaching and dyed by a reactive dye, and then treated by a hydrophilic treatment to give a clothing fabric. The obtained clothing fabric had a total width of 178 cm and a basis weight of 227 g/m$^2$. Next, the electrodes and the wiring were formed on the skin-side surface of the clothing fabric similarly to Example 1.

Example 3

Rovings having a mass of 80 grains/15 yd were produced using a total 100 mass% of cotton (effective fiber length: 41 mm) obtained by blending Suvin cotton (effective fiber length: 42.5 mm) and Giza 45 cotton (effective fiber length: 39 mm) at a mass ratio of 50 : 50. The obtained rovings were drafted at 42 times and real-twisted at the number of twists T: 44.1 times/2.54 cm (twist coefficient K: 5.69) using a ring spinning frame, and thus spun into spun yarns with a yarn count of 60. The number of fluffy fibers having a length of 1 mm or more, per 10 m of the obtained spun yarns, was 403.6 (fibers/10 m). Next, the spun yarns were knitted in interlock stich using 4AL double knitting machine 33"-28G manufactured by Precision Fukuhara Works, Ltd., under the condition of 260 mm/100 W. The obtained knitted gray fabric was subjected to common scouring and bleaching and dyed by a reactive dye, and then treated by a hydrophilic treatment to give a clothing fabric. The obtained clothing fabric had a width of 130 cm and a basis weight of 176 g/m$^2$. Next, the electrodes and the wiring were formed on the skin-side surface of the clothing fabric similarly to Example 1.

Comparative Example 1

Rovings having a mass of 160 grains/15 yd were produced using, as the type of cotton, 100 mass% of cotton used in Example 1. The obtained rovings were drafted at 28 times and real-twisted at the number of twists T: 13.4 times/2.54 cm (twist coefficient K: 3.0) using a ring spinning frame, and thus spun into spun yarns with a yarn count of 20. The number of fluffy fibers having a length of 1 mm or more, per 10 m of the obtained spun yarns, was 1038.5 (fibers/10 m). Next, the spun yarns were knitted in jersey stich using 3FA single knitting machine 34"-22G manufactured by Precision Fukuhara Works, Ltd., under the condition of 350 mm/100 W. The obtained knitted gray fabric was subjected to common scouring and bleaching and dyed by a reactive dye, and then treated by a hydrophilic treatment to give a clothing fabric. The obtained clothing fabric had a width of 155 cm and a basis weight of 270 g/m$^2$. Next, the electrodes and the wiring were formed on the skin-side surface of the clothing fabric similarly to Example 1.

Comparative Example 2

Rovings having a mass of 120 grains/15 yd were produced using 100 mass% of cotton used in Example 1. The obtained rovings were drafted at 42 times and real-twisted at the number of twists T: 23.7 times/2.54 cm (twist coefficient K: 3.75) using a ring spinning frame, and thus spun into spun yarns with a yarn count of 40. The number of fluffy fibers having a length of 1 mm or more, per 10 m of the obtained spun yarns, was 864.8 (fibers/10 m). Next, the spun yarns were knitted in jersey stich using 3FA single knitting machine 34"-28G manufactured by Precision Fukuhara Works, Ltd., under the condition of 250 mm/100 W. The obtained knitted gray fabric was subjected to common scouring and bleaching and dyed by a reactive dye, and then treated by a hydrophilic treatment to give a clothing fabric. The obtained clothing fabric had a width of 180 cm and a basis weight of 225 g/m$^2$. Next, the electrodes and the wiring were formed on the skin-side surface of the clothing fabric similarly to Example 1.

Comparative Example 3

Rovings having a mass of 80 grains/15 yd were produced using 100 mass% of cotton used in Example 2. The obtained rovings were drafted at 42 times and real-twisted at the number of twists T: 28.2 times/2.54 cm (twist coefficient K: 3.64) using a ring spinning frame, and thus spun into spun yarns with a yarn count of 60. The number of fluffy fibers having a length of 1 mm or more, per 10 m of the obtained spun yarns, was 531.2 (fibers/10 m). Next, the spun yarns were knitted in interlock stich using 4AL double knitting machine 33"-28G manufactured by Precision Fukuhara Works, Ltd., under the condition of 260 mm/100 W. The obtained knitted gray fabric was subjected to common scouring and bleaching and dyed by a reactive dye, and then treated by a hydrophilic treatment to give a clothing fabric. The obtained clothing fabric had a width of 140 cm and a basis weight of 168 g/m$^2$. Next, the electrodes and the wiring were formed on the skin-side surface of the clothing fabric similarly to Example 1.

Comparative Example 4

Rovings having a mass of 80 grains/15 yd were produced using a total 100 mass% of polyethylene terephthalate fibers (fineness: 1.3 dtex, semi dull, round cross-section fibers) having a fiber length of 38 mm. The obtained rovings were drafted at 42 times and real-twisted at the number of twists T: 28.2 times/2.54 cm (twist coefficient K: 3.64) using a ring spinning frame, to give spun yarns with a yarn count of 60. The number of fluffy fibers having a length of 1 mm or more, per 10 m of the obtained spun yarns, was 528.5 (fibers/10 m). Next, the spun yarns were knitted in interlock stich using 4AL double knitting machine 33"-28G manufactured by Precision Fukuhara Works, Ltd., under the condition of 260 mm/100 W. The obtained knitted gray fabric was subjected to common scouring and bleaching and dyed by a reactive dye, and then treated by a hydrophilic treatment to give a clothing fabric. The obtained clothing fabric had a width of 138 cm and a basis weight of 172 g/m². Next, the electrodes and the wiring were formed on the skin-side surface of the clothing fabric similarly to Example 1.
Table 1 shows the physical properties and the evaluation results of these clothing fabrics.

[Table 1]

| | | Example 1 | Example 2 | Example 3 | Comparative Example 1 | Comparative Example 2 | Comparative Example 3 | Comparative Example 4 |
|---|---|---|---|---|---|---|---|---|
| Spun yarn | Type | Single yarn | Spun twisted union yarn | Single yarn | Single yarn | Single yarn | Single yarn | Single yarn |
| | Eeffective fiber length of cotton (mm) | 32.5 | 37.5 | 41.0 | 32.5 | 32.5 | 37.5 | 38.0 |
| | Blending proportion of cotton (mass%) | 100 | 100 | 100 | 100 | 100 | 100 | 0 |
| | T : Number of twists (times/2.54 cm) | 17.5 | 23.3 | 44.1 | 13.4 | 23.7 | 28.2 | 28.2 |
| | N e : English yarn count | 20 | 40 | 60 | 20 | 40 | 60 | 60 |
| | F : Number of fluffy fibers (fibers/10 m) having a length of 1 mm or more | 792.6 | 361.7 | 403.6 | 1038.5 | 864.8 | 531.2 | 528.5 |
| | Formula (1):F/(0.8673√Ne) | 4087 | 2638 | 3605 | 5355 | 6306 | 4744 | 4720 |
| | Formula (2): K : Twist coefficient (T/√Ne) | 3.91 | 3.68 | 5.69 | 3.00 | 3.75 | 3.64 | 3.64 |
| Clothing fabric | Structure | Jersey stitch | Jersey stitch | Interlock stitch | Jersey stitch | Jersey stitch | Interlock stitch | Interlock stitch |
| | Total width (cm) | 157 | 178 | 130 | 155 | 180 | 140 | 138 |
| | Basis weight (g/m²) | 264 | 227 | 176 | 270 | 225 | 168 | 172 |

| | | Example 1 | Example 2 | Example 3 | Comparative Example 1 | Comparative Example 2 | Comparative Example 3 | Comparative Example 4 |
|---|---|---|---|---|---|---|---|---|
| Electrode | First insulating layer | Polyurethane hot-melt sheet | Polyurethane hot-melt sheet | Polyurethane hot-melt sheet | Polyurethane hot-melt sheet | Polyurethane hot-melt sheet | Polyurethane hot-melt sheet | Polyurethane hot-melt sheet |
| | Conductive layer | Nitrile rubber 20 (parts by mass) Silver particles 110 (parts by mass) | Nitrile rubber 20 (parts by mass) Silver particles 110 (parts by mass) | Nitrite rubber 20 (parts by mass) Silver particles 110 (parts by mass) | Nitrile rubber 20 (parts by mass) Silver particles 110 (parts by mass) | Nitrile rubber 20 (parts by mass) Silver particles 110 (parts by mass) | Nitrile rubber 20 (parts by mass) Silver particles 110 (parts by mass) | Nitrile rubber 20 (parts by mass) Silver particles 110 (parts by mass) |
| Peel strength (N/cm) | | 8.6 | 11.2 | 7.8 | 6.2 | 4.9 | 6.9 | 6.8 |
| Moisture absorptivity (%) | | 7.5 | 7.5 | 7.6 | 7.5 | 7.4 | 7.5 | 0.4 |
| Pilling (class) | | 4 | 4.5 | 4 | 3.5 | 3 | 3.5 | 2 |
| Frictional electrostatic voltage (V) | | 950 | 820 | 980 | 1350 | 1850 | 1050 | 3850 |

EP 3 951 035 A1

**Claims**

1. A garment comprising:

   a clothing fabric; and
   an electrode formed on a skin-side surface of the clothing fabric,
   the clothing fabric including spun yarns present on the skin-side surface on which the electrode is formed, and
   each of the spun yarns satisfying a following formula (1):

   $$F/(0.8673/\sqrt{Ne}) < 4500 \ (1),$$

   wherein F represents a number of fluffy fibers (fibers/10 m) having a length of 1 mm or more, per 10 m of the spun yarn, and Ne represents an English yarn count of the spun yarn.

2. The garment according to claim 1, having a peel strength between the clothing fabric and the electrode of 7.0 N/cm or more, the peel strength being measured in accordance with JIS L 1086 (2013), 7.10.

3. The garment according to claim 1 or 2, wherein
   each of the spun yarns includes 65 mass% or more of cotton.

4. The garment according to any one of claims 1 to 3, comprising 80 mass% or more of the spun yarns in 100 mass% of the clothing fabric.

5. The garment according to any one of claims 1 to 4, wherein each of the spun yarns is twisted to have a twist coefficient K of 3.20 or more, the twist coefficient K being obtained by a following formula (2):

   $$K = T/\sqrt{Ne} \ (2),$$

   wherein K represents the twist coefficient, T represents a number of twists (times/2.54 cm) applied to the spun yarn, and Ne represents the English yarn count of the spun yarn.

6. The garment according to any one of claims 1 to 5, wherein the spun yarns are spun twisted union yarns.

7. The garment according to any one of claims 1 to 6, wherein the clothing fabric has a basis weight of 150 g/m$^2$ or more and 300 g/m$^2$ or less.

8. The garment according to any one of claims 1 to 7, wherein the clothing fabric is a knitted fabric, includes knitted loops present on the skin-side surface thereof, and has, in the knitted loops, a proportion in number of 50% or more of knitted loops formed of the spun yarns.

9. The garment according to any one of claims 1 to 8, wherein the electrode includes an insulating layer formed on the skin-side surface of the clothing fabric, and a conductive layer formed on the insulating layer.

10. The garment according to any one of claims 1 to 9, being an underwear top.

11. The garment according to any one of claims 1 to 9, being an underwear bottom.

12. The garment according to any one of claims 1 to 9, being a band-shaped fabric.

<div align="center">

**INTERNATIONAL SEARCH REPORT**

</div>

| | International application No. |
|---|---|
| | PCT/JP2020/014442 |

| A. CLASSIFICATION OF SUBJECT MATTER |
|---|
| Int.Cl. D02G3/26(2006.01)i, D04B1/14(2006.01)i, A41B9/06(2006.01)i, A41D13/00(2006.01)i, A41D13/12(2006.01)i, A61B5/0408(2006.01)i, A61B5/0478(2006.01)i, A41D31/04(2019.01)i FI: A41D13/00102, A61B5/04300M, A41D13/12145, A41D13/12154, A41D31/04Z, A41B9/06Z, D04B1/14, D02G3/26 |
| According to International Patent Classification (IPC) or to both national classification and IPC |

| B. FIELDS SEARCHED |
|---|
| Minimum documentation searched (classification system followed by classification symbols) |
| Int.Cl. D02G3/26, D04B1/14, A41B9/06, A41D13/00, A41D13/12, A61B5/0408, A61B5/0478, A41D31/04 |

Documentation searched other than minimum documentation to the extent that such documents are included in the fields searched

| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| Y | JP 2017-29692 A (TOYOBO CO., LTD.) 09.02.2017 (2017-02-09), paragraphs [0022]-[0026] | 1-12 |
| Y | JP 2015-203159 A (TORAY INDUSTRIES, INC.) 16.11.2015 (2015-11-16), paragraphs [0062]-[0068] | 1-4, 7-12 |
| Y | JP 2019-31757 A (INNOVATIVE MARKETLINK KK) 28.02.2019 (2019-02-28), paragraphs [0022], [0026], [0028] | 1-12 |
| Y | JP 2018-53407 A (UNITIKA TRADING CO., LTD.) 05.04.2018 (2018-04-05), paragraph [0019] | 6 |
| Y | JP 2012-102417 A (TOYOBO SPECIALTIES TRADING CO., LTD.) 31.05.2012 (2012-05-31), paragraph [0035] | 8 |

☐ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16.06.2020 | 23.06.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

INTERNATIONAL SEARCH REPORT

Information on patent family members

International application No.

PCT/JP2020/014442

| JP 2017-29692 A | 09.02.2017 | JP 2017-29691 A |
| | | JP 2017-29693 A |
| | | JP 2017-35457 A |
| JP 2015-203159 A | 16.11.2015 | (Family: none) |
| JP 2019-31757 A | 28.02.2019 | (Family: none) |
| JP 2018-53407 A | 05.04.2018 | (Family: none) |
| JP 2012-102417 A | 31.05.2012 | (Family: none) |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017029692 A
- JP 2019067964 A